# FASCICULE DE BREVET EUROPEEN

(11) **EP 1 150 723 B1**
(45) Date de publication et mention de la délivrance du brevet: **11.09.2002**
(21) Numéro de dépôt: 00903761.5
(22) Date de dépôt: 09.02.2000
(51) Int. Cl.: A61L 24/06, C08F 222/10

(54) **MATERIAU DE SUTURE DE PLAIES A BASE DE METHYLIDENE MALONATE**
WUNDNAHTMATERIAL AUF DER BASIS VON METHYLIDENMALONAT
SUTURE MATERIAL FOR WOUNDS BASED ON METHYLIDENE MALONATE

(30) Priorité: 09.02.1999 FR 9901485
(43) Date de publication de la demande: 07.11.2001
(73) Titulaire: Virsol, 75116 Paris (FR)
(72) Inventeur: BRU-MAGNIEZ, Nicole, F-75016 Paris (FR); BRETON, Pascal, F-45510 Tigy (FR); ROQUES-CARMES, Claude, F-25000 Besançon (FR); BELIARD, Isabelle, Toronto, Ontario M5R 2K1 (CA)
(74) Mandataire: Hubert, Philippe
(86) Numéro de dépôt international: FR0000305
(87) Numéro de publication internationale: WO00047242

(56) Documents cités:
- WO-A-96/00760
- WO-A-96/02278
- WO-A-98/18455
- DE-A- 19 508 049
- US-A- 5 575 997
- US-A- 5 624 669

## Description

La présente invention a pour objet un matériau de suture de plaies à base de méthylidène malonate.

Dans le cadre de la présente description, on entend désigner par le terme "matériau de suture", un matériau biocompatible permettant, par adhésion, d'abouter les lèvres des plaies, d'arrêter les saignements (l'hémostase) et de favoriser la cicatrisation des tissus lésés.

L'invention trouve essentiellement application dans le domaine du traitement des plaies épidermiques, dermo-épidermiques ou hypo-dermo-épidermiques, en particulier des plaies dermo-épidermiques franches.

On sait que les médecins disposent actuellement de quatre moyens principaux pour traiter les plaies dermo-épidermiques, à savoir la suture par fil résorbable ou non, la pose d'agrafes, la pose de bandelettes adhésives ou l'utilisation de colles cutanées.

La suture par fil est généralement utilisée pour rapprocher les plans superficiels dermiques et épidermiques, dans le cas des fils non résorbables, ou pour rapprocher les plans profonds musculaires et hypodermiques dans le cas des fils résorbables.

Cette méthode de traitement est actuellement la plus couramment utilisée.

Cependant, sa mise en oeuvre est relativement complexe puisqu'elle nécessite une anesthésie locale, une instrumentation stérile associée (pinces, ciseaux, porte-aiguilles...), des soins infirmiers dans la suite du geste thérapeutique ainsi qu'une ablation des fils après quelques jours.

Si ceci n'est pas trop contraignant dans le cadre d'un geste postopératoire, il n'en est pas de même lors du traitement des plaies post-traumatiques traitées au cabinet du médecin ou dans un service d'urgence.

De plus, ce mode de traitement est plus ou moins traumatisant psychologiquement pour le patient, surtout quand il s'agit d'enfants, pour lesquels un geste rapide et indolore s'impose.

La pose d'agrafes soulève, en pratique, sensiblement les mêmes problèmes que la suture par fil.

La pose de bandelettes adhésives, comme par exemple le produit connu sous la dénomination commerciale Stéri-Strip®, permet de traiter les plaies bénignes d'une façon pratiquement indolore.

Cependant, son utilisation reste limitée au traitement des seules plaies dermo-épidermiques de petite taille (de l'ordre du centimètre), et en dehors de toute zone corporelle susceptible de subir des contraintes mécaniques.

Depuis quelques années, différents matériaux adhésifs bio-compatibles ont été développés pour assurer la suture des plaies.

Ces matériaux adhésifs généralement désignés par le terme de "colles" peuvent être répartis en deux catégories :
- les colles biologiques, généralement synthétisées à partir de protéines plasmatiques ;
- les colles synthétiques, principalement à base de cyanoacrylate, et en particulier, de 2-octylcyanoacrylate, 2-éthylcyanoacrylate, 2-butylcyanoacrylate et 2-isobutylcyanoacrylate.

Les colles biologiques sont des produits choisis pour reproduire la dernière phase de la coagulation et assurer l'ancrage du caillot au tissu via l'apport de fibronectine ("protéine de jonction") sur les sites de cicatrisation.

Ces colles biologiques sont particulièrement avantageuses dans la mesure où elles permettent de resolidariser les tissus lésés et de mettre en place des tissus de cicatrisation.

Elles présentent cependant comme principal inconvénient, le risque sous-jacent de transmission virale.

Les colles synthétiques sont, quant à elles, particulièrement avantageuses dans la mesure où :
- elles se présentent généralement sous une forme prête à l'emploi ;
- elles peuvent être utilisées pour la suture de plaies de tailles plus ou moins importantes (de 1 à 10 cm environ) ; et
- la qualité esthétique des cicatrices après suturation apparaît comme égale ou supérieure à celle des cicatrices obtenues par suture par fil ou par agrafes.

Cependant les colles synthétiques actuellement disponibles sur le marché, présentent de nombreux inconvénients.

En premier lieu, ces colles sont généralement liquides et, étant appliquées exclusivement en surface, elles ont tendance à couler au-delà de leur zone d'application.

En second lieu, ces colles sont généralement potentiellement allergènes et se dégradent très difficilement dans l'organisme en formant généralement des produits considérés comme toxiques pour celui-ci, ce qui en limite considérablement l'intérêt.

Dans ces conditions, la présente invention a pour but de résoudre le problème technique consistant en la fourniture d'un nouveau matériau de suture de plaies, de nature essentiellement synthétique, qui présente les mêmes avantages que les colles synthétiques précitées, qui soit d'une mise en oeuvre aisée, qui puisse se dégrader relativement facilement dans l'organisme sans générer de produits toxiques et qui puisse être appliqué à l'intérieur de la plaie dans toutes les couches cutanées lésées.

La présente invention a encore pour but de résoudre le problème technique précité d'une façon applicable à l'échelle industrielle.

Il a été découvert, et ceci constitue le fondement de la présente invention, que certaines compositions à base de monomères et/ou d'oligomères et/ou de polymères à base de méthylidène malonate présentent l'ensemble des propriétés requises, notamment en terme de bio-adhésion et de viscosité avant et/ou après application, pour réaliser de nouveaux matériaux de suture de plaies répondant à cet objectif.

Ainsi, selon un premier aspect, la présente invention a pour objet un matériau de suture de plaies constitué d'un mélange biocompatible, bio-adhésif comprenant au moins 50 % en poids, et de préférence au moins 80 % en poids, d'une composition à base de méthylidène malonate contenant :
- de 40 à 100 % en poids, et de préférence de 50 à 100 % en poids d'un ou plusieurs méthylidène malonate de formule (I) 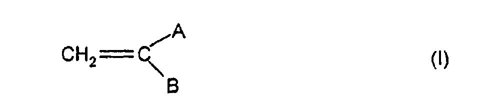 dans laquelle :
   - A et B représentent indépendamment un groupe (a) ou (b): 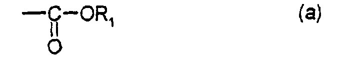 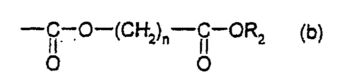
   dans lesquels R₁ et R₂ représentent indépendamment un groupe alkyle linéaire ou ramifié ayant de 1 à 6 atomes de carbone, et n est un nombre entier compris entre 1 et 5; étant précisé que l'un au moins de A et B représente un groupe (b);
et/ou d'un ou plusieurs oligomère(s) de méthylidène malonate présentant un poids moléculaire inférieur ou égal à 6 000 et constitué(s) d'unités récurrentes de formule (II): 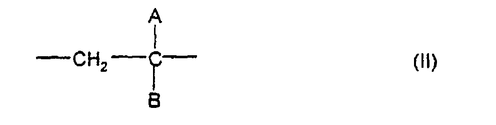 dans laquelle A et B sont tels que définis précédemment ;
- de 0 à 60 % en poids, et de préférence de 0 à 50 % en poids d'un ou plusieurs polymère(s) de méthylidène malonate présentant un poids moléculaire supérieur à 6 000 et constitué(s) d'unités récurrentes de formule (II).

Selon une caractéristique particulière de l'invention, dans les formules (I) et (II) précitées :
- A représente un groupe (a) dans lequel R₁ représente un groupe alkyle ayant de 1 à 6 atomes de carbone, de préférence un groupe éthyle,
- B représente un groupe (b) dans lequel R₂ représente un groupe alkyle ayant de 1 à 6 atomes de carbone, de préférence un groupe éthyle, et n est un nombre égal à 1.

Dans un mode de réalisation préféré de l'invention, le matériau est constitué pour au moins 90 %, et, de préférence pour au moins 95 % en poids de la composition à base de méthylidène malonate précitée, et celle-ci contient:
- de 50 à 90 % en poids d'un ou plusieurs oligomère(s) de méthylidène malonate présentant un poids moléculaire inférieur ou égal à 6.000 de préférence inférieur ou égal à 3.000 et constitué(s) d'unités récurrentes de formule (II),
- de 10 à 50 % en poids d'un ou plusieurs polymères de méthylidène malonate présentant un poids moléculaire supérieur à 6.000,
et le matériau précité présente une température de transition vitreuse inférieure ou égale à 0°C, de préférence comprise entre -10 et -35°C, et de préférence encore entre -20 et -30°C.

Cette composition est nouvelle en tant que telle et la présente demande vise également à la protéger en tant que matériau nouveau.

Dans un mode de réalisation actuellement préféré de l'invention, la composition à base de méthylidène malonate précitée contient :
- de 55 à 65 % en poids d'un ou plusieurs oligomère(s) de méthylidène malonate présentant un poids moléculaire inférieur ou égal à 3.000, et de préférence compris entre 300 et 1000 et constitué(s) d'unités récurrentes de formule (II),
- de 35 à 45 % en poids d'un ou plusieurs polymères de méthylidène malonate présentant un poids moléculaire supérieur à 6.000, de préférence supérieur à 9.000, et de préférence compris entre 12.000 et 25.000.

Ainsi, le matériau de suture de plaies conforme à la présente invention est essentiellement caractérisé par le fait qu'il est constitué majoritairement d'une composition à base de méthylidène malonate elle-même majoritairement constituée de monomère(s) et/ou d'oligomère(s) de poids moléculaire inférieur ou égal à 6000, de préférence inférieur ou égal à 3000.

Une telle composition présente des propriétés de viscosité et de bioadhésion (ou collage) permettant son utilisation dans le traitement des plaies dermo-épidermiques, seule ou en mélange avec d'autres composants biocompatibles.

Il a en particulier été observé qu'une telle composition peut être appliquée à l'intérieur de la plaie, sans qu'aucun saignement persistant ne soit constaté lors du collage, et sans qu'aucune réaction inflammatoire ne soit constatée après 10 jours de collage.

En outre, les compositions à base de méthylidène malonate utilisables dans le cadre de l'invention, sont dégradables par bio-érosion, en libérant de l'éthanol et de l'acide glycolique qui sont généralement considérés comme non toxiques pour l'organisme, l'acide glycolique semblant même agir comme stimulateur de croissance cellulaire.

Ces compositions à base de méthylidène malonate pourront être facilement préparées par l'homme de métier, éventuellement par simple mélange dans un solvant approprié de ses constituants (monomériques, oligomériques, polymériques) préparés séparément, et évaporation subséquente du solvant.

Les monomères de méthylidène malonate pourront être préparés en suivant le procédé décrit dans le brevet EP 0.283.346 correspondant aux brevets US 4.931.584 et US 5.142.098, après dégazage sous vide de pompe à palettes jusqu'à poids constant pour éliminer l'inhibiteur de polymérisation (SO₂).

Les oligomères et polymères de méthylidène malonate pourront être synthétisés par voie anionique ou radicalaire à partir des monomères précités.

Dans le cas des compositions à base de méthylidène malonate préférées, qui sont formées d'un mélange d'oligomère(s) et de polymère(s), ces compositions pourront être obtenues également en une seule étape ; les proportions relatives des constituants pouvant être ajustées en faisant varier la concentration en amorceur anionique ou radicalaire dans le milieu de polymérisation.

Les caractéristiques physico-chimiques des compositions à base de méthylidène malonate précitées pourront être facilement ajustées par l'homme de métier, pour obtenir un matériau de suture de plaies présentant les caractéristiques de bio-adhésion et de viscosité requises.

Comme on le comprend, ces caractéristiques physico-chimiques devront être ajustées en fonction de la nature de l'ensemble des composants du matériau de suture, l'objectif étant d'obtenir un matériau bio-adhésif et de viscosité appropriée.

D'une façon générale, les constituants du matériau de suture de plaies conforme à l'invention, autres que la composition à base de méthylidène malonate précitée, pourront représenter jusqu'à 50 % en poids de ce matériau.

Ces constituants seront bien entendu choisis de telle sorte à former, avec les compositions à base de méthylidène malonate précitées, des mélanges intimes présentant les caractéristiques de bio-adhésion et de viscosité recherchées.

De préférence, lorsqu'ils seront présents, ces constituants ne représenteront qu'environ 1 à 20 %, de préférence encore 1 à 10 % en poids, rapporté au poids total du matériau de suture.

Ces constituants pourront être de nature variée, d'origine naturelle ou synthétique.

A titre d'exemple de tels constituants, on citera notamment :
- les dextranes solubles ou insolubles substitués fonctionnalisés qui sont notamment décrits dans les brevets FR n° 2.555.589 et FR n° 2.461.724 :

- les polycyanoacrylates, de préférence les polyalkylcyanoacrylates ;
- les polyméthacrylates d'alkyle ;
- les polyuréthanes biocompatibles ;
- les polyoxyalkylènes ;
- les polyaminoacides ;
- les polylactates ;
- les polylactate-co-glycolates ;
- les polyalcoolvinyliques.

Des constituants additionnels préférés sont par exemple le polyéthylène glycol, additif hydrophile appartenant à la famille des polyoxyalkylènes susceptible de jouer le rôle de plastifiant au sein du mélange, ou bien encore le poly(lactide-co-glycolide), additif biodégradable appartenant à la famille des polylactate-co-glycolates susceptible de permettre une amélioration de la biodégradabilité du mélange.

D'une façon générale, ces constituants seront présents au sein du matériau de suture sous forme de mélanges avec les compositions à base de méthylidène malonate précitées.

Il convient de noter que, sans sortir du cadre de la présente invention, ces constituants pourront également être présents au sein du matériau de suture sous forme d'unités monomériques dans des copolymères comprenant des unités méthylidène malonate de formule (II), telle que définie précédemment.

Ces copolymères à base de méthylidène malonate pourront être préparés par les techniques de polymérisation classiques bien connues de l'homme de métier, parmi lesquelles on citera la polymérisation par voie anionique, la polymérisation par voie radicalaire ou encore la technique de couplage des séquences précurseurs du copolymère, ces séquences ayant été au préalable fonctionnalisées en bout de chaîne de façon adéquate.

D'une façon générale, les unités monomériques formant les constituants précités seront choisies parmi les unités monomériques constitutives des polyacrylates, des polysaccharides, des polyoxyalkylènes, des polylactates et des polylactate-co-glycolates.

Parmi les unités monomériques constitutives de polysaccharides susceptibles d'être utilisés dans le cadre de l'invention, on citera plus particulièrement les unités monomériques constitutives des dextranes solubles ou insolubles substitués fonctionnalisés qui sont notamment décrits dans les brevets FR n° 2.555.589 et FR n° 2.461.724.

Parmi les unités monomériques constitutives de polyacrylates susceptibles d'être utilisées dans le cadre de l'invention, on citera les alkylcyanoacrylates, les méthacrylates d'alkyle et les itaconates.

D'une façon générale, les copolymères à base de méthylidène malonate utilisés dans le cadre de la présente invention seront constitués, pour au moins 50 % de leurs unités monomériques, par des unités méthylidène malonate.

Ces copolymères pourront être statistiques ou présenter des structures à blocs ou greffées.

Le matériau de suture conforme à la présente invention peut, le cas échéant, comprendre parmi ses constituants, des produits biocompatibles susceptibles d'ajuster sa viscosité, comme par exemple des plastifiants ou bien encore des produits biocompatibles permettant d'améliorer l'adhésion, comme par exemple des résines dites "tackifiantes".

Parmi les agents plastifiants qui conviennent dans le cadre de la présente invention, on citera par exemple les esters dérivés de l'acide adipique, de l'acide azélaïque, de l'acide citrique, de l'acide oléique, de l'acide stéarique, de l'acide sébaccique ; le polyéthylène glycol.

Parmi les résines tackifiantes qui conviennent dans le cadre de la présente invention, on peut mentionner les résines polyterpènes ou terpènes modifiées, les résines hydrocarbonées, les mélanges de résines aromatiques et aliphatiques.

Le matériau de suture, conforme à la présente invention, peut en outre comprendre parmi ses constituants un ou plusieurs principes actifs choisis notamment parmi les anesthésiques locaux, comme la lidocaïne ; les agents bactériostatiques et les agents antibiotiques, comme par exemple la streptomycine ; les antalgiques, comme par exemple le kétoprofène.

Les matériaux de suture conforme à la présente invention trouvent application dans le domaine du traitement de plaies épidermiques, dermo-épidermiques, hypo-dermo-épidermiques, et peuvent être appliqués à l'intérieur de ladite plaie, le matériau de suture de plaies étant préalablement préchauffé le cas échéant à une température supérieure à sa température de ramollissement.

Dans le cas des matériaux préférés constitués essentiellement de compositions à base de méthylidène malonate formées d'un mélange d'oligomères et de polymères présentant une température de transition vitreuse comprise entre 0 et -50°C, la température de préchauffage précitée est de l'ordre de 35°C à 47°C.

D'autres matériaux préférés conforme à la présente invention sont indiqués dans les revendications.

Comme on le comprend, le matériau de suture conforme à la présente invention, sera conditionné sous une forme permettant son application, comme par exemple à l'intérieur d'une seringue auto-chauffante.

La présente invention va maintenant être illustrée par les exemples non limitatifs suivants.

Dans ces exemples, les abréviations suivantes ont été utilisées :
MM 2.1.2: méthylidène malonate répondant à la formule : 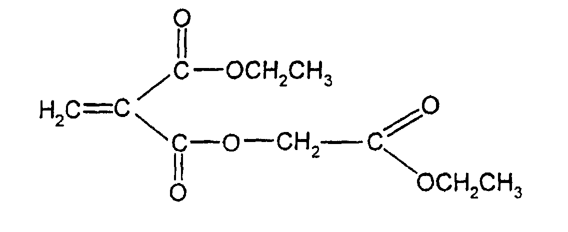 encore dénommé : 1-éthoxycarbonyl-1-éthoxycarbonylméthylène-oxycarbonyl éthène
PMM 2.1.2: oligomère ou polymère constitué d'unités monomères récurrentes répondant à la formule 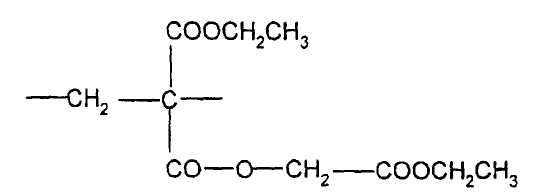
PEG : poly(éthylèneglycol)
PLGA : poly(lactide-co-glycolide)
POE : poly(oxyéthylène)
PεCL : poly(ε caprolactone)
DCC : dicyclohexylcarbodiimide

De même, dans ces exemples ainsi que dans la description, on entend par "poids moléculaire", la masse molaire moyenne en poids désignée M_{w} , exprimée en g/mole d'équivalent polystyrène (PS), et mesurée par la méthode de Chromatographie par Perméation de Gel (CPG) avec un appareillage chromatographique étalonné avec des polymères étalons de polystyrène.

Par ailleurs, la température de transition vitreuse (Tg) a été déterminée par analyse enthalpique différentielle avec une vitesse de balayage de 10°C par minute.

### EXEMPLE 1

### Préparation d'un matériau de suture de plaies conforme à l'invention

### A. Protocole expérimental

Différentes compositions à base de méthylidène malonate formées de mélanges d'oligomères et de polymères ont été préparées par polymérisation anionique (NaOH 0,1N) en milieu solvant (acétone) à partir du monomère, conformément au mode opératoire suivant :
- dans un ballon de 250 ml, une quantité m (exprimée en grammes) de monomère, maintenu sous vide primaire pendant 5 heures afin d'éliminer l'inhibiteur de polymérisation (dioxyde de soufre) et dissout dans un volume V (exprimé en millilitres) d'acétone est maintenue sous agitation magnétique pendant 10 minutes.
- puis un volume V' (exprimé en millilitres) de soude 0,1 N est additionné en une seule étape, toujours sous agitation magnétique.

L'agitation est maintenue pendant 15 minutes environ, puis la polymérisation est éventuellement stoppée (si celle-ci n'est pas déjà achevée) par ajout d'acide chlorhydrique décimolaire en un volume sensiblement identique au volume de soude, l'acétone est évaporée sous vide et le polymère ainsi obtenu est lavé avec de l'eau distillée puis séché sur gel de silice.

Alternativement, il est possible de préparer ces compositions en ajoutant la solution de monomères dans l'acétone à une solution de soude également dans l'acétone.

### B. Caractéristiques des compositions préparées

On a mentionné dans le tableau I ci-après les conditions expérimentales utilisées pour la préparation de cinq compositions à base de méthylidène malonate ainsi que la température de transition vitreuse (Tg) de ces compositions.

**TABLEAU I**

| Composition n° | m (g) MM2.1.2 | V (ml) d'acétone | V' (ml) NaOH | Tg (°C) |
|---|---|---|---|---|
| 1 | 1 | 50 | 10 | - 22 |
| 2 | 3 | 150 | 30 | - 20 |
| 3 | 1 | 50 | 2,6 | - 13 |
| 4 | 1 | 50 | 2,6 | - 11 |
| 5 | 3 | 150 | 3 | - 10 |

La composition n°1 est constituée pour environ 80 % en poids d'oligomères de poids moléculaire inférieur à 3 000 (Mw=600 environ), et pour environ 20 % en poids de polymères dont le représentant majoritaire a un poids moléculaire de 10 500.

La composition n°2 est constituée pour environ 78 % en poids d'oligomères de poids moléculaire inférieur à 3 100, et pour environ 22 % en poids de polymères dont le représentant majoritaire a un poids moléculaire de 13 000.

La composition n°5 est constituée pour environ 78 % en poids d'oligomères de poids moléculaire inférieur à 5 100 et d'environ 22 % en poids de polymères dont le représentant majoritaire a un poids moléculaire d'environ 18 000.

Une étude menée sur des lambeaux de peau humaine provenant de déchets opératoires sur abdominoplasties a permis de confirmer que toutes ces compositions présentent l'ensemble des qualités requises, notamment en terme de bio-adhésion et de viscosité, pour pouvoir être utilisées comme matériau de suture, seules ou en association avec d'autres constituants bio-compatibles, tels que définis précédemment.

En particulier, il a été observé que le pouvoir collant des cinq compositions à base de méthylidène malonate précitées est stable à 24 heures sans qu'aucune différence ne soit mise en évidence entre des plaies collées et des plaies collées-stripées.

En outre, il a été observé qu'une faible quantité de composition est suffisante pour obtenir un pouvoir collant satisfaisant lorsqu'une compression manuelle des deux berges de la plaie est réalisée après application de ladite composition.

Les meilleurs résultats ont été obtenus avec la composition n°1.

Lorsque de telles compositions sont destinées à être utilisées seules, il est nécessaire que la température de transition vitreuse soit inférieure à 0°C, pour éviter que ces compositions ne prennent en masse trop rapidement avant l'application à l'intérieur de la plaie.

Avantageusement, cette température de transition vitreuse sera comprise entre - 10 et - 35°C, et de préférence encore entre - 20°C et - 30°C.

### Préparation d'autres matériaux de suture de plaie conforme à l'invention

### A/ Protocole expérimental

Différentes compositions à base de méthylidène malonate formées de mélange d'oligomères et de polymères et incorporant éventuellement un ou plusieurs autres constituants ont été préparées par mélange dans un solvant approprié.

Plus précisément, les oligomères de méthylidène malonate, les polymères de méthylidène malonate et éventuellement un constituant additionnel ont été pesés dans des quantités préalablement déterminées, et solubilisées dans un solvant commun (généralement l'acétone) sous agitation magnétique, puis le solvant a été évaporé à l'aide d'un évaporateur rotatif, et le mélange séché dans un dessicateur sous vide primaire.

### B/ Caractéristiques des compositions préparées

On a mentionné dans le Tableau IA ci-après les principales caractéristiques des compositions ainsi préparées.

Les compositions n° 6 et 7 sont uniquement constituées d'oligomères et de polymères de méthylidène malonate 2.1.2.

Les compositions n° 8 et 9 comportent en outre un additif hydrophile du type PEG de faible masse molaire qui peut être utilisé en tant que plastifiant au sein du mélange.

Les compositions n° 10 et 11 comportent en outre un additif bio-dégradable du type PLGA qui peut être utilisé pour moduler la bio-dégradabilité du mélange.

Le PLGA utilisé présente les caractéristiques suivantes :
Mw = 50000-70000 ; Tg=45-50°C ; (Aldrich 43,044-7)

La composition n° 11 comporte en outre une partie constituée de monomères de méthylidène malonate 2.1.2, ce monomère ayant été ajouté à froid à un mélange dans l'acétone constitué des trois autres composants.

Les compositions n° 12 et 13 comportent en outre un additif amphiphile, lui-même à base de méthylidène malonate 2.1.2 qui présente une bonne affinité avec les autres composants et permet d'améliorer la tenue de la colle sur la plaie.

Cet additif amphiphile est un copolymère à blocs présentant 250 motifs d'oxyde d'éthylène et 43 motifs MM 2.1.2 préparés par voie anionique par polymérisation successive de l'oxyde d'éthylène puis du MM 2.1.2.

La composition n° 14 comporte en outre un additif bio-dégradable à base de MM 2.1.2 qui est un copolymère PMM 2.1.2 - PεCL qui présente une bonne affinité avec le mélange et une séquence biodégradable permettant de moduler la cinétique de dégradabilité globale dudit mélange.

Cet additif est un copolymère à blocs présentant une séquence PMM 2.1.2 de 5800 g/mol, et une séquence PεCL de 2000 g/mol qui a été préparé par couplage chimique entre les deux homopolymères, le PMM 2.1.2 α-hydroxy fonctionnalisé et la PεCLα-carboxy fonctionnalisée, en présence de DCC dans le dichlorométhane.

**TABLEAU IA**

| Composition No. | Mw* oligomères du MM2.1.2. (g/mol) | Mw* PMM2.1.2. (g/mol) | Nature additif | Composition massique oligomères/ PMM2.1.2./ additifs | Tg du mélange (°C) |
|---|---|---|---|---|---|
| 6 | 1950 | 29 300 | | 91/9 | -26.9 |
| 7 | 700 | 15000 | | 60/40 | -15 |
| 8 | 1950 | - | PEG750 | 95/0/5 | -22.3 |
| 9 | 1950 | 34 700 | PEG550 | 80/16/4 | -31.3 |
| 10 | 2200 | - | PLGA | 95/0/5 | -34.2 |
| 11 | 1450 | 60 500 | MM2.1.2./PLGA | 60/30/5/5 | -41.7 |
| 12 | 2200 | - | POE-PMM2.1.2. | 95/0/5 | -45.1 |
| 13 | 1450 | 60 500 | MM2.1.2./POE-PMM2.1.2. | 60/30/5/5 | -44.4 |
| 14 | 1450 | | PεCL-PMM2.1.2 | 91/9 | -22.7 |

| | | | | | |
|---|---|---|---|---|---|
| * équivalent PS | | | | | |

### EXEMPLE 2

### Mise en évidence de l'intérêt des compositions à base méthylidène malonate en tant que constituant essentiel d'un matériau de suture de plaies

Le pouvoir collant des compositions à base de méthylidène malonate a été confirmé par la réalisation d'une étude in vivo sur des cicatrices dermo-épidermiques chez le cobaye.

Cette étude a été réalisée avec la composition n° 1 précitée qui présente une température de transition vitreuse de -22°C, et en parallèle contre un système de référence (suture par fil non résorbable).

Le protocole suivant a été mis en oeuvre.

Les animaux retenus sont des cobayes (n = 10) Hartley mâles d'un poids de 250-300 g (Charles Rivers, France) exempts de toute pathologie virale, bactérienne, fongique et parasitaire.

Tous les cobayes ont été préalablement rasés après une période d'acclimatation de trois jours dans des cages à lapins P 3 000 (IFFA CREDO, France).

Cinq d'entre eux sont tirés au sort et subissent :
a - une désinfection à la Bétadine moussante pour un second rasage (rasage de près final),
b - une anesthésie générale de 3 minutes à l'Halothane (3 %) avec un débit d'oxygène de 3 l.min⁻¹ (ACOMA VAPORIZER®, Japon),
c - une seconde désinfection complémentaire à la Bétadine jaune,
d - une incision dermo-épidermique paravertébrale de 1 cm de long environ, avec un bistouri à lame n°15 stérile (Swann-Morton, Angleterre). Le muscle peaucier sert de repère pour les plans anatomiques,
e - une application immédiate à l'intérieur de la plaie en un seul mouvement lent d'une extrémité à l'autre de la plaie de la composition testée, celle-ci ayant été préchauffé dans une coupelle en verre sur une plaque chauffante réglée à 47°C. La température est prise dans un ballon témoin contenant de l'eau. Dès la fin de l'incision, un prélèvement de la composition testée est effectué dans la coupelle à l'aide d'une spatule fine de chimiste (3 mm de large et 1 mm d'épaisseur) en vue de l'application,
f - un rapprochement digital des 2 berges de la plaie pendant 30 secondes, et un retrait du surplus superficiel de la composition testée à l'aide d'une compresse,
g - une pose de 3 bandes adhésives (Stéri-strip).

Les cinq cobayes tirés au sort restants composent le groupe témoin ; ils subissent une incision suivie d'une suture.

Le protocole opératoire retenu est identique à celui décrit précédemment, si ce n'est qu'à partir de l'étape "e", chaque cobaye est suturé à l'aide d'un monofil Prolène Bleu 4/0 muni d'une aiguille courbe P3, section de 13mm.

Le mode de suture de l'incision est le suivant :
- cobaye N°6 : 2 points de suture,
- cobaye N°7 : 2 points de suture,
- cobaye N°8 : 3 points de suture,
- cobaye N°9 : 2 points de suture,
- cobaye N°10: 3 points de suture.

Dès la fin de l'opération, chaque cobaye est remis dans sa cage, se réveille en moins de 5 minutes après l'opération, puis est libre de tout mouvement.

Les plaies sont désinfectées localement et quotidiennement à la Bétadine jaune. Des clichés photographiques des plaies sont réalisés à T = 1 j ; T = 3 j et T = 10 j.

Après six jours, a lieu l'ablation des fils sous anesthésie générale.

Une évaluation de la qualité esthétique des cicatrices est réalisée en aveugle par un médecin urgentiste habitué à la pratique et au suivi des sutures, à l'aide d'une échelle analogique visuelle. Les résultats entre les deux groupes sont comparés à l'aide d'un test non paramétrique de Mann et Whitney unidirectionnel à T = 10 j (le seuil de signification est fixé à 5 %).

Les cobayes sont sacrifiés par injection intra-péritonéale d'une solution de phénobarbital après onze jours pour le groupe "colle" et après douze jours pour le groupe "suture".

Deux exérèse-biopsies, par cobaye, sont pratiquées : une témoin en peau saine et une prenant de façon globale la cicatrice.

Les prélèvements sont mis en solution dans du formol à 10% en attente de l'analyse anatomo-pathologique.

Les résultats suivants ont été obtenus.

La durée totale des opérations a été de 1 heure entre le premier et le dernier cobaye dans chaque groupe.

Plusieurs constatations ont été effectuées :
a- à T = 1 j, le cobaye n°6 a perdu spontanément ses trois fils et les cobayes n°8 et n°10 ont perdu un fil de façon spontanée à T = 5 j.
Les bandes adhésives de tous les cobayes du groupe colle (composition n°1) sont parties spontanément au bout de 24 heures,
b - aucune infection locale ni générale n'est à déplorer,
c - d'un point de vue tactile en aveugle, les cicatrices sur polymères sont reconnues comme étant douces et non rugueuses à l'inverse des cicatrices sur fil,
d - la qualité esthétique de chaque plaie à T = 10 j a été évaluée en aveugle à l'aide d'une échelle analogique visuelle (0-200) et les résultats obtenus sont reportés au tableau II.

**TABLEAU II**

| Cobaye n = 5 | Composition n° 1 (score) | | Cobaye n = 5 | Suture fil (score) |
|---|---|---|---|---|
| 1 | 125 | | 6 | 141 |
| 2 | 190 | | 7 | 121 |
| 3 | 196 | | 8 | 180 |
| 4 | 168 | | 9 | 90 |
| 5 | 179 | | 10 | 80 |
| Médiane | 179 | p = 0,0476 | | 121 |
| minimum | 125 | | | 80 |
| maximum | 196 | | | 180 |

L'analyse statistique portant sur la qualité esthétique des cicatrices (échelle analogique visuelle) après ablation des fils au 10^{ème} jour, met en évidence une différence statistiquement significative (p = 0,0476) en faveur de la composition à base de méthylidène malonate par rapport au fil de suture.

Aucune infection n'est à déplorer parmi les deux groupes au cours de l'étude.

La même étude in vivo a été réalisée avec les compositions n° 6, 8, 9, 10, 11, 12, 13 et 14.

Le protocole opératoire est identique à celui décrit ci-dessus, le mode de suture de l'incision étant dans tous les cas pour le groupe témoin de 3 points de suture.

Le Tableau III ci-après donne les résultats relatifs à la qualité esthétique de chaque plaie à T=21 j, évaluée en aveugle à l'aide d'une échelle analogique visuelle (0-200).

L'analyse statistique de ces résultats met en évidence une différence statistiquement significative (p=0,0005) en faveur des compositions à base de méthylidène malonate par rapport au fil de suture.

Les cicatrices par fil sont toujours pénalisées par un aspect en "barreaux d'échelle".

**TABLEAU III**

| COMPOSITION No. | QUALITE ESTHETIQUE DE LA PLAIE COLLEE (score/200) | QUALITE ESTHETIQUE DE LA PLAIE SUTUREE (score/200) |
|---|---|---|
| 6 | 192 | 175 |
| 8 | 189 | 168 |
| 9 | 184 | 155 |
| 10 | 183 | 162 |
| 11 | 175 | 172 |
| 12 | 178 | 155 |
| 13 | 183 | 169 |
| 14 | 169 | 160 |

Une étude anatomo-pathologique a été réalisée :
- d'une part pour vérifier les risques inflammatoires éventuels dus aux produits de dégradation du polyméthylidène malonate,
- d'autre part pour contrôler le caractère biorésorbable de la composition à base de méthylidène malonate utilisée.

Les résultats obtenus montrent que, sur un plan histologique, les compositions à base de méthylidène malonate ont un comportement tout à fait satisfaisant.

## Revendications

1. Matériau de suture de plaies, **caractérisé en ce qu'**il est constitué d'un mélange biocompatible, bio-adhésif comprenant au moins 50 % en poids, et de préférence au moins 80 % en poids. d'une composition à base de méthylidène malonate contenant :
- de 40 à 100% en poids, et de préférence de 50 à 100 % en poids d'un ou plusieurs méthylidène malonate de formule (I) 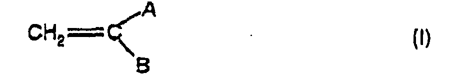 dans laquelle :
- A et B représentent indépendamment un groupe (a) ou (b): 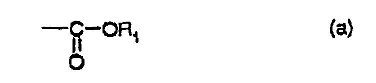 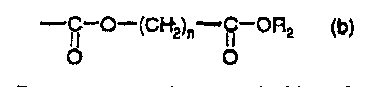 dans lesquels R₁ et R₂ représentent indépendamment un groupe alkyle linéaire ou ramifié ayant de 1 à 6 atomes de carbone, et n est un nombre entier compris entre 1 et 5, étant précisé que l'un au moins de A et B représente un groupe (b) ;
et/ou d'un ou plusieurs oligomère(s) de méthylidène malonate présentant un poids moléculaire inférieur ou égal à 6 000 et constitué(s) d'unités récurrentes de formule (II): 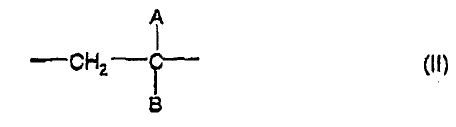 dans laquelle A et B sont tels que définis précédemment ;
- de 0 à 60 % en poids, et de préférence de 0 à 50 % en poids d'un ou plusieurs polymcre(s) de méthylidène malonate présentant un poids moléculaire supérieur à 6 000 et constitué(s) d'unités récurrentes de formule (II)

2. Matériau selon la revendication 1, **caractérisé en ce que** dans les formules (I) et (II) précitées :
- A représente un groupe (a) dans lequel R₁ représente un groupe alkyle ayant de 1 à 6 atomes de carbone, de préférence un groupe éthyle,
- B représente un groupe (b) dans lequel R₂ représente un groupe alkyle ayant de 1 à 6 atomes de carbone, de préférence un groupe éthyle, et n est un nombre égal à 1.

3. Matériau selon la revendication 2, **caractérisé en ce que** dans les formules (I) et (II) précitées :
- A représente un groupe (a) dans lequel R₁ représente un groupe éthyle ; et
- B représente un groupe (b) dans lequel R₂ représente un groupe éthyle et n est un nombre égal à 1.

4. Matériau selon l'une des revendications 1 à 3, **caractérisé en ce que** la composition à base de méthylidène malonate précitée contient :
- de 50 à 90 % en poids d'un ou plusieurs oligomère(s) de méthylidène malonate présentant un poids moléculaire inférieur ou égal à 6.000 de préférence inférieur ou égal à 3.000 et constitué(s) d'unités récurrentes de formule (II).
- de 10 à 50 % en poids d'un ou plusieurs polymères de méthylidène malonate présentant un poids moléculaire supérieur à 6.000,
et **en ce que** ledit matériau présente une température de transition vitreuse inférieure ou égale à 0°C, de préférence comprise entre -10 et -35°C.

5. Matériau selon la revendication 4, **caractérisé en ce que** la composition à base de méthylidène malonate précitée contient :
- de 55 à 65 % en poids d'un ou plusieurs oligomère(s) de méthylidène malonate présentant un poids moléculaire inférieur ou égal à 3,000 et constitué(s) d'unités récurrentes de formule (II),
- de 35 à 45 % en poids d'un ou plusieurs polymères de méthylidène malonate présentant un poids moléculaire supérieur à 6.000, de préférence supérieur à 9.000.

6. Matériau selon la revendication 5, **caractérisé en ce que** la composition à base de méthylidène malonate précitée contient :
- de 55 à 65 % en poids d'un ou plusieurs oligomère(s) de méthylidène malonate présentant un poids moléculaire compris entre 300 et 1000 et constitué(s) d'unités récurrentes de formule (II),
- de 35 à 45 % en poids d'un ou plusieurs polymères de méthylidène malonate présentant un poids moléculaire compris entre 12.000 et 25.000.

7. Matériau selon l'une des revendications 4 à 6, **caractérisé en ce qu'**il est constitué pour au moins 90 %, et de préférence pour au moins 95 % en poids d'une composition à base de méthylidène malonate telle que définie à l'une des revendications 4 à 6.

8. Matériau selon l'une des revendications 4 à 6, **caractérisé en ce qu'**il comprend jusqu'à 50 % en poids, et de préférence jusqu'à 20 % en poids d'un ou plusieurs constituant(s) autre(s) que la composition à base de méthylidène malonate précitée, choisi(s) parmi:
- les polycyanoacrylates, de préférence les polyalkylcyanoacrylates ;
- les polyméthacrylates d'alkyle ;
- les polyuréthanes biocompatibles ;
- les polyoxyalkylènes ;
- les polyaminoaeides ;
- les polylactates ;
- les polylactate-co-glycolates ;
- les polyalcoolvinyliques.

9. Matériau selon la revendication 8, **caractérisé en ce que**, au moins pour partie, le(s) constituants) précité(s) est(sont) présent(s) sous forme d'unités monomériques associées à des unités méthylidène malonate de formule (II) précitée, dans des copolymères, et en particulier sous forme d'unités monvmérigues constitutives des polyacrylates, des polysaccharides, des polyoxyalkylènes, des polylactates et des polylactate-co-glycolates.

10. Matériau, **caractérisé en ce qu'**il est constitué pour au moins 90%, et de préférence pour au moins 95% en poids d'une composition à base de méthylidène malonate contenant :
- de 50 à 90 % en poids d'un ou plusieurs oligomère(s) de méthylidène malonate présentant un poids moléculaire inférieur ou égal à 6.000 de préférence inférieur ou égal à 3.000 et constitué(s) d'unités récurrentes de formule (II),
- de 10 à 50 % en poids d'un ou plusieurs polymères de méthylidène malonate présentant un poids moléculaire supérieur à 6.000,
et **en ce que** ledit matériau présente une température de transition vitreuse inférieure ou égale à 0°C, de préférence comprise entre -10 et -35°C.

11. Matériau selon la revendication 10, **caractérisé en ce que** la composition à base de méthylidène malonate précitée contient :
- de 55 à 65 % en poids d'un ou plusieurs oligomère(s) de méthylidène malonate présentant un poids moléculaire inférieur ou égal à 3.000 et constitué(s) d'unités récurrentes de formule (II),
- de 35 à 45 % en poids d'un ou plusieurs polymères de méthylidène malonate présentant un poids moléculaire supérieur à 6.000, de préférence supérieur à 9.000.

12. Matériau selon la revendication 11, **caractérisé en ce que** la composition à base de méthylidène malonate précitée contient :
- de 55 à 65 % en poids d'un ou plusieurs oligomère(s) de méthylidène malonate présentant un poids moléculaire compris entre 300 et 1000 et constitué(s) d'unités récurrentes de formule (II),
- de 35 à 45 % en poids d'un ou plusieurs polymères de méthylidène malonate présentant un poids moléculaire compris entre 12.000 et 25.000.

## Patentansprüche

1. Wundnahtmaterial, **dadurch gekennzeichnet, dass** es aus einer biokompatiblen, bio-adhäsiven Mischung besteht, die mindestens 50 Gew.%, und bevorzugt mindestens 80 Gew.% einer Zusammensetzung auf Grundlage von Methylidenmalonat umfasst, enthaltend:
- 40 bis 100 Gew.%, und bevorzugt 50 bis 100 Gew.% von einem oder mehreren Methylidenmalonat(en) der Formel. (I) 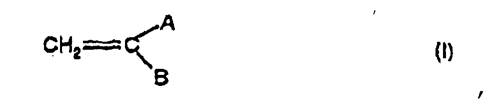
wobei
- A und B unabhängig voneinander eine Gruppe (a) oder (b) darstellen: 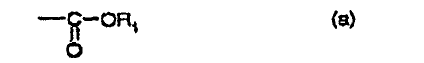 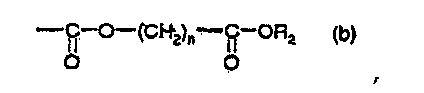 in denen R₁ und R₂ unabhängig voneinander eine lineare oder verzweigte Alkylgruppe mit 1 bis 6 Kohlenstoffatomen darstellen, und n eine ganze Zahl zwischen 1 und 5 ist, wobei mindestens einer von A und B die Gruppe (b) darstellt;
und/oder von einem oder mehreren Methylidenmalonatoligomore(n), die ein Molekulargewicht unterhalb von oder gleich 6.000 aufweisen und aus den sich wiederholenden Einheiten der Formel (II) besteht/bestehen: 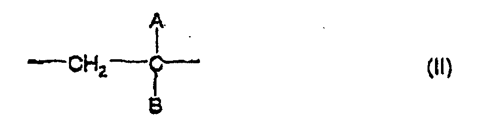 wobei A und B wie zuvor definiert sind;
- 0 bis 60 Gew.%, und bevorzugt 0 bis 50 Gew.% von einem oder mehreren Methylidenmalonatpolymer(en), das/die ein Molekulargewicht über 6.000 aufweist/aufweisen und aus den sich wiederholenden Einheiten der Formel (II) beteht/bestehen.

2. Material gemäß Anspruch 1, **dadurch gekennzeichnet, dass** in den oben erwähnten Formeln (I) und (II):
- A eine Gruppe (a) darstellt, in der R₁ eine Alkylgruppe mit 1 bis 6 Kohlenstoffatomen, bevorzugt eine Ethylgruppe, darstellt,
- B eine Gruppe (b) darstellt, in der R₂ eine Alkylgruppe mit 1 bis 6 Kohlenstoffatomen darstellt, bevorzugt eine Ethylgruppe, und n eine Zahl ist, die gleich 1 ist.

3. Material gemäß Anspruch 2, **dadurch gekennzeichnet, dass** in den zuvor erwähnten Formeln (I) und (II):
- A eine Gruppe (a) darstellt, in der R₁ eine Ethylgruppe darstellt; und
- B eine Gruppe (b) darstellt, in der R₂ eine Ethylgruppe darstellt und n eine Zahl ist, die gleich 1 ist.

4. Material gemäß einem der Ansprüche 1 bis 3, **dadurch gekennzeichnet, dass** die oben erwähnte Zusammensetzung auf Grundlage von Methylidenmalonat:
- 50 bis 90 Gew.% von einem oder mehreren Methylidenmalonatoligomer(en) mit einem Molekulargewicht unter oder gleich 6.000, bevorzugt unterhalb von oder gleich 3.000 und aus sich wiederholenden Einheiten der Formel (II) bestehend,
- 10 bis 50 Gew.% von einem oder mehreren Methylidenmalonatpolymer(en), das/die ein Molekulargewicht über 6000 besitzt/besitzen, enthält,
und dadurch, dass das Material eine Glasumwandlungstemperatur unterhalb von oder gleich 0°C, bevorzugt zwischen -10 und -35°C, aufweist.

5. Material gemäß Anspruch 4, **dadurch gekennzeichnet, dass** die oben erwähnte Zusammensetzung auf Grundlage von Methylidenmalonat:
- 55 bis 65 Gew.% von einem oder mehreren Methylidenmalonatoligomer(en), das/die ein Molekulargewicht unter oder gleich 3.000 aufweist/aufweisen und aus wiederholten Einheiten der Formel (II) besteht/bestehen,
- 35 bis 45 Gew.% von einem oder mehreren Methylidenmalonatoligomer(en), das/die ein Molekulargewicht über 6.000, bevorzugt über 9.000 aufweist/aufweisen, enthält.

6. Material gemäß Anspruch 5, **dadurch gekennzeichnet, dass** die oben erwähnte Zusammensetzung auf Grundlage von Methylidenmalonat:
- 55 bis 65 Gew.% von einem oder mehreren Methylidenmalonatoligomer(en), das/die ein Molekulargewicht zwischen 300 und 1.000 aufweist/aufweisen, und aus wiederholten Einheiten der Formel (II) bestehen,
- 35 bis 45 Gew.% von einem oder mehreren Methylidenmalonatoligomer(en), das/die ein Molekulargewicht zwischen 12.000 und 25.000 aufweist/aufweisen, enthält.

7. Material gemäß einem der Ansprüche 4 bis 6, **dadurch gekennzeichnet, dass** es aus mindestens 90 Gew.% und bevorzugt mindestens 95 Gew.% einer Zusammensetzung auf Grundlage von Methylidenmalonat, wie in einem der Ansprüche 4 bis 6 definiert, besteht.

8. Material gemäß einem der Ansprüche 4 bis 6, **dadurch gekennzeichnet, dass** es bis 50 Gew.%, und bevorzugt bis zu 20 Gew.% von einem oder mehreren Bestandteil(en), neben der oben erwähnten Zusammensetzung auf Grundlage des Methylidenmalonats umfasst, das/die ausgewählt ist/sind aus:
- Polycyanoacrylaten, bevorzugt Polyalkylcyanoacrylaten;
- Polyalkylmethacrylaten;
- biokompatiblen Polyurethanen;
- Polyoxyalkylenen;
- Polyaminosäuren;
- Polylactaten;
- Polylactat-co-glykolaten;
- Polyvinylalkoholen.

9. Material gemäß Anspruch 8, **dadurch gekennzeichnet, dass** der(die) Bestandteil(e), der(die) oben erwähnt wurde(n), zumindestens zum Teil in Form von Monomereinheiten, die mit den Methylidenmalonateinheiten der oben erwähnten Formel (II) in Copolymeren verbunden sind, und insbesondere in Form von konstitutiven Monomereinheiten aus Polyacrylaten, Polysacchariden, Polyoxyalkylenen, Polylactaten und Polylactat-co-glykolaten.

10. Material, **dadurch gekennzeichnet, dass** es mindestens aus 90 Gew.%, bevorzugt mindestens aus 95 Gew.% einer Zusammensetzung auf Grundlage von Methylidenmalonat besteht, die
- das 50 bis 90 Gew.% von einem oder mehreren Methylidenmalonatoligomer(en) mit einem Molekulargewicht unterhalb von oder gleich 6.000, bevorzugt unterhalb von oder gleich 3.000, das/die aus sich wiederholenden Einheiten der Formel (II) gebildet werden,
- 10 bis 50 Gew.% von einem oder mehreren Methylidenmalonatoligomer(en) mit einem Molekulargewicht über 6.000 enthält,
und dadurch, dass das Material eine Glasumwandlungstemperatur unterhalb von oder gleich 0°C, bevorzugt zwischen -10 und -35°C aufweist.

11. Material gemäß Anspruch 10, **dadurch gekennzeichnet, dass** die oben erwähnte Zusammensetzung auf Grundlage des Methylidenmalonats
- 55 bis 65 Gew.% von einem oder mehreren Methylidenmalonatoligomer (en) mit einem Molekulargewicht unter oder gleich 3.000, und sich wiederholenden Einheiten der Formel (II) bestehen,
- 35 bis 45 Gew.% von einem oder mehreren Methylidenmalonatoligomer (en) mit einem Molekulargewicht über 6.000, bevorzugt über 9.000, enthält.

12. Material gemäß Anspruch 11, **dadurch gekennzeichnet, dass** die oben erwähnte Zusammensetzung auf Grundlage des Methylidenmalonats
- 55 bis 65 Gew.% von einem oder mehreren Methylidenmalonatoligomer(en) mit einem Molekulargewicht zwischen 300 und 1.000 aufweist und aus wiederholten Einheiten der Formel (II) besteht,
- 35 bis 45 Gew.% von einem oder mehreren Methylidenmalonatoligomer(en) mit einem Molekulargewicht zwischen 12.000 und 25.000 enthält.

## Claims

1. Material for the suture of wounds, **characterised in that** it consists of a biocompatible, bio-adhesive mixture comprising at least 50% by weight, and preferably at least 80% by weight, of a methylidene malonate-based composition containing:
- 40 to 100% by weight, and preferably 50 to 100% by weight of one or more methylidene malonates of formula (I) 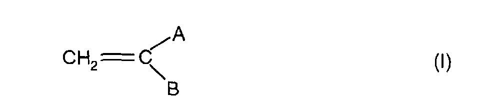 in which:
- A and B independently represent a group (a) or (b): 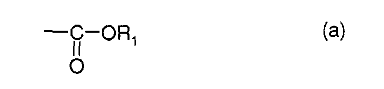 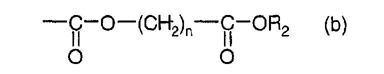 in which R₁ and R₂ independently represent a linear or branched alkyl group with 1 to 6 carbon atoms, and n is an integer between 1 and 5;
and/or one or more methylidene malonate oligomers with a molecular weight less than or equal to 6,000 and consisting of recurrent units of formula (II): 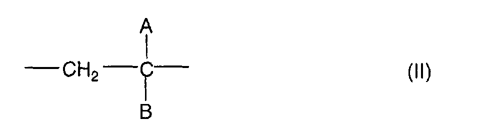 in which A and B are as defined above;
- 0 to 60% by weight, and preferably 0 to 50% by weight of one or more methylidene malonate polymers with a molecular weight greater than 6,000 and consisting of recurrent units of formula (II).

2. Material according to claim 1, **characterised in that** in aforementioned formulae (I) and (II):
- A represents a group (a) in which R₁ represents an alkyl group with 1 to 6 carbon atoms, preferably an ethyl group,
- B represents a group (b) in which R₂ represents an alkyl group with 1 to 6 carbon atoms, preferably an ethyl group, and n is a number equal to 1.

3. Material according to claim 2, **characterised in that** in aforementioned formulae (I) and (II):
- A represents a group (a) in which R₁ represents an ethyl group; and
- B represents a group (b) in which R₂ represents an ethyl group and n is a number equal to 1.

4. Material according to one of claims 1 to 3, **characterised in that** the aforementioned methylidene malonate-based composition contains:
- 50 to 90% by weight of one or more methylidene malonate oligomers with a molecular weight less than or equal to 6,000, preferably less than or equal to 3,000 and consisting of recurrent units of formula (II),
- 10 to 50% by weight of one or more methylidene malonate polymers with a molecular weight greater than 6,000,
and **in that** the said material has a glass transition temperature less than or equal to 0°C, preferably between -10 and -35°C.

5. Material according to claim 4, **characterised in that** the aforementioned methylidene malonate-based composition contains:
- 55 to 65% by weight of one or more methylidene malonate oligomers with a molecular weight that less than or equal to 3,000 and consisting of recurrent units of formula (II),
- 35 to 45% by weight of one or more methylidene malonate polymers with a molecular weight greater than 6,000, preferably greater than 9,000.

6. Material according to claim 5, **characterised in that** the aforementioned methylidene malonate-based composition contains:
- 55 to 65% by weight of one or more methylidene malonate oligomers with a molecular weight between 300 and 1,000 and consisting of recurrent units of formula (II),
- 35 to 45% by weight of one or more methylidene malonate polymers with a molecular weight between 12,000 and 25,000.

7. Material according to one of claims 4 to 6, **characterised in that** it consists of at least 90%, and preferably of at least 95% by weight of a methylidene malonate-based composition as defined in one of claims 4 to 6.

8. Material according to one of claims 4 to 7, **characterised in that** it consists of up to 50% by weight, and preferably up to 20% by weight of one or more constituents other than the aforementioned methylidene malonate-based composition, chosen from:
- polycyanoacrylates, preferably polyalkylcyanoacrylates;
- polyalkylmethacrylates;
- biocompatible polyurethanes;
- polyoxyalkylenes;
- polyaminoacids;
- polylactates;
- polylactate-co-glycolates;
- polyvinylalcohols.

9. Material according to claim 8, **characterised in that** at least in part, the aforementioned constituent(s) is (are) present in the form of monomer units associated with the aforementioned methylidene malonate units of formula (II), in copolymers, and in particular in the form of monomer units forming polyacrylates, polysaccharides, polyoxyalkylenes, polylactates and polylactate-co-glycolates.

10. Material, **characterised in that** it consists of at least 90%, and preferably of at least 95% by weight of a methylidene malonate composition containing:
- 50 to 90% by weight of one or more methylidene malonate oligomers with a molecular weight less than or equal to 6,000, preferably less than or equal to 3,000 and consisting of recurrent units of formula (II),
- 10 to 50% by weight of one or more methylidene malonate polymers with a molecular weight greater than 6,000,
and **in that** the said material has a glass transition temperature less than or equal to 0°C, preferably between -10 and -35°C.

11. Material according to claim 10, **characterised in that** the aforementioned methylidene malonate-based composition contains:
- 55 to 65% by weight of one or more methylidene malonate oligomers with a molecular weight that less than or equal to 3,000 and consisting of recurrent units of formula (II),
- 35 to 45% by weight of one or more methylidene malonate polymers with a molecular weight greater than 6,000, preferably greater than 9,000.

12. Material according to claim 11, **characterised in that** the aforementioned methylidene malonate-based composition contains:
- 55 to 65% by weight of one or more methylidene malonate oligomers with a molecular weight between 300 and 1,000 and consisting of recurrent units of formula (II),
- 35 to 45% by weight of one or more methylidene malonate polymers with a molecular weight between 12,000 and 25,000.
